# EUROPÄISCHE PATENTSCHRIFT

(11) **EP 3 108 226 B1**
(45) Veröffentlichungstag und Bekanntmachung des Hinweises auf die Patenterteilung: **27.07.2022**
(21) Anmeldenummer: 15706153.2
(22) Anmeldetag: 11.02.2015
(51) Int. Cl.: G01N 27/404, G01N 33/00

(54) **ELEKTROCHEMISCHER GASSENSOR**
ELECTROCHEMICAL GAS SENSOR
DÉTECTEUR DE GAZ ÉLECTROCHIMIQUE

(30) Priorität: 21.02.2014 DE 102014002500
(43) Veröffentlichungstag der Anmeldung: 28.12.2016
(73) Patentinhaber: Dräger Safety AG & Co. KGaA, 23560 Lübeck (DE)
(72) Erfinder: PILZ, Sabrina, 23558 Lübeck (DE); METT, Frank, 23556 Lübeck (DE)
(74) Vertreter: Guthöhrlein, Gerhard
(86) Internationale Anmeldenummer: PCT/EP2015/000292
(87) Internationale Veröffentlichungsnummer: WO 2015/124276

(56) Entgegenhaltungen:
- EP-A1- 0 152 636
- WO-A1-2010/063626
- DE-A1- 10 048 240
- DE-A1- 19 755 506
- DE-A1-102008 044 239
- DE-A1-102010 021 975
- US-A- 3 305 469
- US-A- 4 662 996
- US-A- 4 666 565

## Beschreibung

Die Erfindung betrifft einen elektrochemischen Gassensor entsprechend dem Oberbegriff von Anspruch 1, insbesondere einen elektrochemischen Gassensor, der zum Nachweis von sauren Gasen und/oder Gasgemischen mit sauren Gasen verwendbar ist.

Elektrochemische Gassensoren sind allgemein bekannt. Sie weisen üblicherweise mehrere Elektroden auf, die in leitendem Kontakt mit einer Elektrolytflüssigkeit stehen und auf diese Weise ein galvanisches Element - im Folgenden auch elektrochemische Messzelle genannt - bilden. Dabei gibt es sowohl Sensoren, die stationär verwendet werden, als auch Sensoren, die in portablen Geräten zum Einsatz kommen.

Das technische Einsatzgebiet solcher Sensoren erstreckt sich zum Beispiel von der chemischen Industrie über die Überwachung von Kühlanlagen bis hin zu landwirtschaftlichen Betrieben. Dabei dienen die Sensoren insbesondere dazu, kritische Konzentrationen von entzündlichen und/oder giftigen Gasen rechtzeitig zu erkennen und vor einer entsprechenden Gefahr zu warnen. In vielen Gebieten der chemischen Industrie ist in diesem Zusammenhang - ebenso wie in der Halbleiterindustrie - insbesondere der Nachweis sogenannter saurer Gase von großem Interesse, um die Arbeitsplatzsicherheit zu gewährleisten. Unter sauren Gasen werden dabei im Sinne der vorliegenden Erfindung allgemein solche Gase verstanden, die bei Lösung in Wasser (schwache) Säuren bilden, beispielsweise Halogen-Wasserstoffe wie etwa HF und HCl, Schwefelwasserstoff oder auch Essigsäure.

In diesem Zusammenhang offenbart GB 2129562 B den elektrochemischen Nachweis von Fluorwasserstoff (HF). Sowohl die Kathode als auch die Anode des dort vorgeschlagenen Sensors sind Platindrähte. Als Elektrolyt wird ein Gemisch aus Calciumbromid und Calciumbromat verwendet. Diese Nachweismethode ist jedoch besonders flow- und temperaturabhängig.

Eine alternative Lösung ist aus WO 2002/031485 A1 bekannt. Hier wird eine Messelektrode aus einem elektrochemisch aktiven Metalloxidpulver vorgeschlagen. Solche Metalloxidpulver können jedoch hohe Querempfindlichkeiten auf andere Gase aufweisen, wodurch sich geringe Konzentrationen des Zielgases nicht immer sicher nachweisen lassen.

Auch WO 1999/001758 A1 offenbart einen elektrochemischen Sensor, der insbesondere zum Nachweis von Chlorwasserstoff verwendet werden soll. Bei diesem Sensor weist die Arbeitselektrode eine elektrochemisch aktive Oberfläche aus Gold auf. Diese löst sich jedoch mit der Zeit auf, was im Ernstfall zum Ausfall des Sensors führen kann.

US 4,662,966 A offenbart eine elektrochemische Methode und Vorrichtung zum Nachweis polarer, toxischer Verbindungen, wobei die Vorrichtung eine polymerbeschichtete Elektrode verwendet.

US 4,666,565 A offenbart eine elektrochemische Zelle, bei der die Arbeitselektrode aus einem absorbierenden Material besteht.

DE 10 2010 021 975 A1 offenbart einen elektrochemischen Gassensor zum Nachweis von Blausäure, der Lithiumbromid in wässriger Lösung aufweist.

US 3,305,469 A offenbart eine elektrochemische Zelle, die zum Nachweis von gasförmigen Boranen in der Umgebungsluft geeignet ist und bei der Elektrode Silbercarbonat aufweist.

DE 197 55 506 A1 offenbart einen elektrochemischen Sensor mit offenem Gehäuse, bei dem Adsorptionsmittelreservoir vorgesehen ist.

Ausgehend davon ist es Aufgabe der vorliegenden Erfindung, diese und weitere Nachteile des Standes der Technik zu überwinden und einen verbesserten elektrochemischen Gassensor bereit zu stellen. Insbesondere soll ein Gassensor bereitgestellt werden, der eine möglichst hohe Messempfindlichkeit und eine möglichst gute Signalstabilität bei Dauerbelastung aufweist. Weiterhin soll der Gassensor möglichst kostengünstig und einfach herstellbar sein.

Als Lösung sieht die Erfindung einen elektrochemischen Gassensor mit den Merkmalen des Anspruchs 1, sowie die Verwendung eines solchen Gassensors gemäß Anspruch 12 vor. Weitere Ausgestaltungen sind Gegenstand der abhängigen Ansprüche.

Insbesondere sieht die Erfindung einen elektrochemischen Gassensor mit einem Gehäuse, mit mehreren darin angeordneten Elektroden, nämlich wenigstens einer Arbeitselektrode und einer Gegenelektrode, sowie mit einem flüssigen Elektrolyten, wobei wenigstens die Gegenelektrode und/oder das Gehäuse zumindest teilweise aus einer Absorptionsmittelzusammensetzung besteht, vor, wobei die Absorptionsmittelzusammensetzung BaCO₃ als Absorptionsmittel enthält, wobei das Gehäuse eine Ausnehmnung aufweist, die einen Gaseinlass bildet, und eine Ausnehmung aufweist, die einen Gasauslass bildet, wobei die Absorptionsmittelzusammensetzung ganz oder teilweise in der Ausnehmung angeordnet ist und wobei die Absorptionsmittelzusammensetzung, die in der Ausnehmung angeordnet ist die Form eines Stopfens aufweist.

Als Gehäuse wird in diesem Zusammenhang typischerweise der Teil des Gassensors angesehen, der den Gassensor nach außen begrenzt. Das Gehäuse steht dabei üblicherweise nach außen hin mit der Umgebungsluft in Kontakt und bildet innen eine Aufnahme für den Elektrolyten und die Elektroden.

Unter einer Absorptionsmittelzusammensetzung wird eine Zusammensetzung verstanden, die wenigstens ein Absorptionsmittel aufweist, wobei das Absorptionsmittel derart ausgebildet ist, dass es mit wenigstens einem an der Gegenelektrode entstehenden Reaktionsprodukt reagieren kann. Eine Absorptionsmittelzusammensetzung im Sinne der vorliegenden Erfindung kann mithin im einfachsten Fall ausschließlich aus einem Absorptionsmittel bestehen. Bevorzugt enthält die Absorptionsmittelzusammensetzung jedoch neben dem Absorptionsmittel noch weitere Inhaltsstoffe, wie nachfolgend näher beschrieben.

Grundsätzlich diffundiert das zu analysierende Gas bei einem solchen elektrochemischen Sensor zunächst an die Arbeitselektrode. Dort wird es reduziert. Die dabei entstehenden Reaktionsprodukte wandern als Ionen zur Gegenelektrode, wo sie wieder oxidiert werden (Rückreaktion).

In dem Gehäuse folgt das zu analysierende Gas mithin einem Diffusionsweg, in dessen Verlauf es zunächst aus der Umgebungsluft in den Elektrolyten gelangt und an dessen Ende die an der Gegenelektrode gebildeten Reaktionsprodukte wieder aus dem Gehäuse heraus strömen. Im einfachsten Fall weist das Gehäuse wenigstens eine Öffnung zum Gasaustausch mit der Umgebung und einen Elektrolyt-gefüllten Reaktionsraum, in welchem sich die Elektroden befinden, auf. Der Diffusionsweg des zu analysierenden Gases führt dann beispielsweise durch die Öffnung in den Reaktionsraum hinein. Im Reaktionsraum strömt das im Elektrolyten gelöste zu analysierende Gas zur Arbeitselektrode, an welcher die Hinreaktion stattfindet. Gleichzeitig diffundiert an der Gegenelektrode während der Rückreaktion gebildetes, ebenfalls im Elektrolyten gelöstes Gas zurück zur Öffnung und von dort aus dem Sensor hinaus. Denkbar ist auch, dass das Gehäuse einen Gaseinlass und einen Gasauslass aufweist, wobei der Diffusionsweg vom Gaseinlass zur Arbeitselektrode und von der Gegenelektrode zum Gasauslass führt.

Ist ein Absorptionsmittel vorgesehen, so kann Gas, das bei der Rückreaktion gebildet wird, absorbieren werden. Auf diese Weise kann sowohl verhindert werden, dass dieses Gas sich an der Gegenelektrode ansammelt, als auch, dass dieses Gas an die Umwelt abgegeben wird.

Die Absorptionsmittelzusammensetzung kann dabei insbesondere solche Reaktionsprodukte absorbieren, die bei der Reaktion an der Gegenelektrode entstehen. Auf diese Weise können beispielsweise Signaleinbrüche verhindert werden, die andernfalls durch die Aufkonzentration dieser Reaktionsprodukte an der Gegenelektrode entstehen könnten. Die Absorptionsmittelzusammensetzung ist dabei derart im Gassensor angeordnet, dass an der Gegenelektrode entstehende Reaktionsprodukte mit der Absorptionsmittelzusammensetzung in Kontakt kommen, wenn sie sich entlang des oben beschriebenen Diffusionsweges im Gassensor bewegen.

Beispielsweise ist denkbar, dass eine der Elektroden aus der Absorptionsmittelzusammensetzung besteht. Vorstellbar ist auch, dass eine der Elektroden einen oder mehrere Abschnitte aufweist, der bzw. die aus der

Absorptionsmittelzusammensetzung bestehen. Mit anderen Worten, es ist denkbar, dass wenigstens eine der Elektroden eine Absorptionsmittelzusammensetzung aufweist.

Besonders günstig ist es, wenn die Absorptionsmittelzusammensetzung an oder in der Gegenelektrode angeordnet ist. Dabei ist beispielsweise vorstellbar, dass die Gegenelektrode aus der Absorptionsmittelzusammensetzung besteht. Die an der Gegenelektrode entstehenden Reaktionsprodukte können auf diese Weise direkt an der Gegenelektrode abgefangen werden.

Beispielsweise ist vorstellbar, dass bei der Hinreaktion an der Arbeitselektrode zunächst Fluorid entsteht, welches an der Gegenelektrode zu HF reagiert. Die Freisetzung von HF aus dem Gassensor ist jedoch in der Regel nicht gewünscht, da HF hochgiftig und ätzend ist. Zudem kann das freigesetzte HF zu der oben beschriebenen Sensordrift führen, wenn es sich im Sensor ansammelt. Vorteilhaft ist es daher wenn das an der Gegenelektrode gebildete HF mit dem in der Absorptionsmittelzusammensetzung der Gegenelektrode enthaltenen Absorptionsmittel reagieren kann. Beispielsweise ist vorstellbar, dass das HF bei dieser Reaktion in einen ausfallenden Feststoff umgesetzt wird.

Vorstellbar ist auch, dass wenigstens ein Abschnitt des Gehäuses aus der Absorptionsmittelzusammensetzung besteht. Bevorzugt ist dabei ein Abschnitt des Gehäuses aus der Absorptionsmittelzusammensetzung gebildet, der in Kontakt mit dem Elektrolyt steht.

Auf diese Weise kann beispielsweise an der Gegenelektrode gebildetes HF oder auch ein anderes Reaktionsprodukt aus dem Elektrolyt abgefangen werden bevor es in die Umgebung des Gassensors freigesetzt wird. So ist es beispielsweise günstig, wenn das Gehäuse eine Ausnehmung aufweist, die einen Gasauslass bildet, wobei die Absorptionsmittelzusammensetzung ganz oder teilweise in der Ausnehmung angeordnet ist.

Beispielsweise kann die Absorptionsmittelzusammensetzung stopfenartig im Gasauslass angeordnet sein. Die Absorptionsmittelzusammensetzung kann dabei einen Filter bilden, durch den das aus dem Gassensor ausströmende Gas hindurch strömen muss. Dabei kann das in der Absorptionsmittelzusammensetzung enthaltene Absorptionsmittel mit dem Reaktionsprodukt reagieren, so dass eine Freisetzung an die Umwelt effektiv verhinderbar ist. Gleichzeitig kann die Absorptionsmittelzusammensetzung dabei eine Außenbegrenzung des Elektrolyt-gefüllten Reaktionsraumes bilden.

Vorstellbar ist dabei, dass entweder eine oder mehrere der Elektroden aus der Absorptionsmittelzusammensetzung bestehen, oder dass ein Teil des Gehäuses, beispielsweise der Gasauslass, aus der Absorptionsmittelzusammensetzung besteht. Auch ist denkbar, dass sowohl wenigstens eine der Elektroden als auch ein Teil des Gehäuses aus der Absorptionsmittelzusammensetzung besteht. Weiterhin ist denkbar, dass eine oder mehrere der Elektroden aus einer ersten Absorptionsmittelzusammensetzung besteht bzw. bestehen während ein Teil des Gehäuses aus einer zweiten Absorptionsmittelzusammensetzung besteht.

Es ist günstig, wenn die Absorptionsmittelzusammensetzung wenigstens ein Absorptionsmittel, ein Trägermaterial und einen Zusatzstoff aufweist. Das Absorptionsmittel ist dabei bevorzugt ein Stoff oder Stoffgemisch, das mit wenigstens einem an der Gegenelektrode entstehenden Reaktionsprodukt reagieren kann. Dabei kann die Wahl der jeweiligen konkreten Zusammensetzung zum einen davon abhängen, welche Reaktionsprodukte an der Gegenelektrode zu erwarten sind, zum anderen davon, ob die Absorptionsmittelzusammensetzung als Elektrodenmaterial und/oder als Bestandteil des Gehäuses, wie beispielsweise als Filter im Gasauslass, verwendet werden soll. In jedem Fall kann die Absorptionsmittelzusammensetzung dabei ein Materialverbund aus Absorptionsmittel, Trägermaterial und Zusatzstoff sein.

Wird die Absorptionsmittelzusammensetzung als Elektrodenmaterial verwendet, so enthält sie zweckmäßigerweise sowohl Material, das im Folgenden als aktives Elektrodenmaterial bezeichnet wird, als auch Material, das im Folgenden als passives Elektrodenmaterial bezeichnet wird. Unter einem aktiven Elektrodenmaterial wird dabei ein Material verstanden, welches elektrochemisch aktiv wirkt und an der elektrochemischen Reaktion des Gassensors beteiligt ist, die an der jeweiligen Elektrode stattfindet. Unter einem passiven Elektrodenmaterial wird in diesem Zusammenhang ein Material verstanden, das nicht an der elektrochemischen Reaktion im eigentlichen Sinne teilnimmt. Unter der elektrochemische Reaktion im eigentlichen Sinne wird die Reaktion zwischen dem zu analysierenden Gas und der Arbeitselektrode bzw. die Rückreaktion an der Gegenelektrode verstanden. Beispielsweise kann der Zusatzstoff der Absorptionsmittelzusammensetzung ein aktives Elektrodenmaterial sein. Vorstellbar ist etwa, dass das aktive Elektrodenmaterial aus Kohlenstoff besteht. Selbstverständlich können jedoch auch andere Materialien, zum Beispiel Metalle bevorzugt Edelmetalle wie bspw. Platin, Iridium oder Gold, als aktives Elektrodenmaterial verwendet werden. In einer bevorzugten Ausführungsform ist es vorgesehen, dass die Absorptionsmittelzusammensetzung Kohlenstoffnanoröhren als Zusatzstoff aufweist. Die Kohlenstoffnanoröhren dienen dabei als aktives Elektrodenmaterial und stellen die elektrische Leitfähigkeit sowie die Reaktionsfähigkeit der Elektrode sicher.

Das Trägermaterial und/oder das Absorptionsmittel können passive Elektrodenmaterialien sein.

Insbesondere wenn die Absorptionsmittelzusammensetzung als Elektrodenmaterial verwendet werden soll, ist es außerdem günstig, wenn die Absorptionsmittelzusammensetzung ein Absorptionsmittel enthält, das schwer löslich oder unlöslich in dem Elektrolyt ist. Bevorzugt ist dabei, wenn das Absorptionsmittel schwer löslich oder besonders bevorzugt unlöslich in dem Elektrolyten ist. Auf diese Weise kann verhindert werden, dass sich die Absorptionsmittelzusammensetzung und mithin das Elektrodenmaterial nach und nach auflöst. Für das entstehende Reaktionsprodukt sollte hingegen eine minimale Löslichkeit vorhanden sein, so dass das Reaktionsprodukt nicht gänzlich unlöslich ist, sondern bevorzugt nur schwer löslich ist. Auf diese Weise kann sich die Elektrodenoberfläche durch Lösen des Reaktionsprodukts in geringem Maße kontinuierlich erneuern. Das dabei stattfindende langsame aber stetige Auflösen des Absorptionsmittels geschieht dabei so langsam und über einen so langen Zeitraum, dass der Verbrauch des Elektrodenmaterials in Bezug auf die Gesamtlebensdauer des Gassensors idealerweise keinen Ausschlag gibt. Gleichzeitig führt die langsame aber stetige Oberflächenerneuerung dazu, dass ein Zusetzen der ElektrodenOberfläche mit Reaktionsprodukten verhinderbar ist.

Weiterhin ist es günstig, wenn die Absorptionsmittelzusammensetzung eine Carbonatverbindung, bevorzugt eine Alkalicarbonatverbindung oder eine Erdalkalicarbonatverbindung, besonders bevorzugt BaCO₃, als Absorptionsmittel enthält. Dies ist vor allem dann vorteilhaft, wenn die nachzuweisenden Gase saure Gase, wie oben definiert, sind. Beispielsweise kann an der Gegenelektrode gebildetes HF mit BaCO₃ zu BaF₂ und H₂CO₃ entsprechend folgender Reaktionsgleichung reagieren. Dabei fällt das gebildete BaF₂ als Feststoff aus:

2HF + BaCO₃ → BaF₂↓ + H₂CO₃

Dabei lagert sich das ausfallende BaF₂ zwar an bzw. in der Gegenelektrode an, jedoch ohne die Gegenelektrode zu vergiften (d.h. ohne die Oberfläche der Elektrode zuzusetzen und damit für weitere Reaktionen zu versiegeln).

Günstig ist es dabei auch, wenn die Absorptionsmittelzusammensetzung ein faserförmiges Material, bevorzugt ein mikrofaserförmiges Material, besonders bevorzugt Glasfasern, Mikro- und/oder Nanofasern, bevorzugt Polymer-Mikro- und/oder Nanofasern, als Trägermaterial enthält. Auf diesem Trägermaterial kann das in der Absorptionsmittelzusammensetzung enthaltene Absorptionsmittel aufgebracht sein. Beispielsweise ist vorstellbar, dass das Trägermaterial mit dem Absorptionsmittel beschichtet ist. Besonders günstig ist es jedoch, wenn die Absorptionsmittelzusammensetzung eine Mischung aus zerkleinertem Trägermaterial und pulverförmigem Absorptionsmittel sowie dem Zusatzstoff ist. Diese Mischung kann beispielsweise zunächst als breiige Masse ähnlich einer Pulpe hergestellt und anschließend in die gewünschte Form - beispielsweise als Elektrode, Filter oder Stopfen für den Gasauslass - gebracht werden. Das Trägermaterial stellt dabei sicher, dass die Mischung aus dem Absorptionsmittel und dem jeweiligen Zusatzstoff einerseits einen wirksamen Verbund bilden kann und andererseits formbar genug ist, um in die gewünschte Form gebracht zu werden. In einer ersten Ausführungsform kann es sich bei dem Trägermaterial um Glasfasermaterial handeln. Vorstellbar ist auch, dass es sich um Nano- oder Mikrofasern bzw. einen Verbund aus Nano- und Mikrofasern aus einem Polymer oder einem Polymergemisch handelt, beispielsweise um elektrogesponnene oder schmelzgesponnene Nanofasern, um Nanofaservliese, um Mikrofasern oder Mikrofaservliese oder auch um zerkleinerte Verbundvliese aus Nano- und Mikrofasern handelt.

Soll die Absorptionsmittelzusammensetzung hingegen ausschließlich als Material im Bereich des Gehäuses verwendet werden, so kann es günstig sein, wenn die Absorptionsmittelzusammensetzung Polytetrafluorethylen (PTFE) oder ein PTFE-derivat als Zusatzstoff aufweist. Dieser Zusatzstoff kann die Diffusion von Gas durch die Absorptionsmittelzusammensetzung erleichtern und damit verbessern. Beispielsweise ist vorstellbar, dass es sich bei dem PTFE oder PTFE-derivat um PTFE-fasern handelt.

Zum Nachweis von sauren Gasen, insbesondere Halogenwasserstoff-Gasen ist es weiterhin vorteilhaft, wenn der Elektrolyt eine Zusammensetzung ist, die ein organisches Lösungsmittel und ein Leitsalz enthält, bevorzugt eine Zusammensetzung, die ein organisches Lösungsmittel, welches ein chinoides System enthält, und ein Leitsalz, welches ein organisches Kation aufweist, enthält. Beispielsweise kann das organische Lösungsmittel ausgewählt sein aus der Gruppe enthaltend Alkylencarbonat, Alkylencarbonat-Gemisch und/oder Butyrolacton, bevorzugt aus der Gruppe enthaltend Propylencarbonat, Ethylencarbonat oder einer Mischung aus Propylen- und Ethylencarbonat. Besonders bevorzugt ist es, wenn das organische Lösungsmittel Sulfolan ist.

Das Leitsalz kann beispielsweise eine ionische Flüssigkeit sein. Bevorzugt ist das Anion des Leitsalzes dabei ausgewählt aus der Gruppe enthaltend Halogenide, Carbonat, Sulfonat, Phosphat und/oder Phosphonat, bevorzugt ein Anion ausgewählt aus der Gruppe enthaltend Alkyl-sulfonat, Alkenyl-Sulfonat, Aryl-Sulfonat, Alkyl-phosphat, Alkenyl-Phosphat, Aryl-Phosphat, substituiertes Alkyl-sulfonat, substituiertes Alkenyl-sulfonat, substituiertes Aryl-sulfonat, substituiertes Alkyl-phosphat, substituiertes Alkenyl-phosphat, substituiertes Aryl-phosphat, halogeniertes Phosphat, halogeniertes Sulfonat halogeniertes Alkyl-sulfonat, halogeniertes Alkenyl-sulfonat, halogeniertes Aryl-sulfonat, halogeniertes Alkyl-phosphat, halogeniertes Alkenyl-phosphat, halogeniertes Aryl-phosphat, besonders bevorzugt ein Anion ausgewählt aus der Gruppe enthaltend Fluorophosphat, Alkylfluorophosphat, Aryl-sulfonat, ganz besonders bevorzugt aus der Gruppe enthaltend Perfluoralkylfluorophosphat, Toluolsulfonat.

Dabei ist es vorteilhaft, wenn das Leitsalz als Kationen Metallionen, Oniumionen oder ein Gemisch aus Metallionen und Oniumionen enthält. Zum Beispiel können die Metallionen ausgewählt sein aus Alkalimetallionen oder Erdalkalimetallionen, bevorzugt aus Li, K und/oder Na. Günstig ist es, wenn die Oniumionen ausgewählt sind aus Ammonium-, Phosphonium, Guanidiniumkationen und heterocyclischen Kationen, bevorzugt ausgewählt aus AlkylAmmonium- und heterocyclischen Kationen, besonders bevorzugt ausgewählt aus AlkylAmmonium, Imidazolium und/oder substituierten Imidazoliumionen, wobei die substituierten Imidazolium-Ionen bevorzugt eine Struktur entsprechend 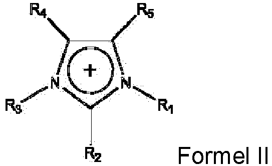 aufweisen, wobei R1, R2, R3, R4 und R5 unabhängig voneinander ausgewählt sein können aus -H, geradkettiges oder verzweigtes Alkyl mit 1 bis 20 C-Atomen, geradkettiges oder verzweigtes Alkenyl mit 2 bis 20 C-Atomen und einer oder mehreren Doppelbindungen, geradkettiges oder verzweigtes Aklinyl mit 2 bis 20 C-Atomen und einer oder mehreren Dreifachbindungen, gesättigtes, teilweise oder vollständig ungesättigtes Cycloalkyl mit 3-7 C-Atomen, das mit Alkylgruppen mit 1 bis 6 C-Atomen substituiert sein kann, gesättigtes teilweise oder vollständig ungesättigtes Heteroaryl, Heteroaryl-C1-C6-alkyl oder Aryl-C1-C6-alkyl, wobei besonders bevorzugt R2, R4 und R5 H sind und R1 und R3 jeweils unabhängig voneinander ein geradkettiges oder verzweigtes Alkyl mit 1 bis 20 C-Atomen.

Beispielsweise ist insbesondere vorstellbar, dass als Leitsalz Tetrabutlyammoniumtoluolsulfonat oder 1-Hexyl-3-methyl-imidazolium-tris(pentafluoroethyl)-trifluorophosphat verwendet wird. So ist es insbesondere vorteilhaft, wenn der Elektrolyt ein Gemisch aus einem Lösungsmittel, einem Leitsalz und/oder einem organischen Mediator ist, , Alkylammonium-toluolsulfonat und ionischen Flüssigkeiten, mit einem Perfluoralkylfluorophosphat-Anion.

Weiterhin ist es günstig, wenn der organische Mediator eine Polyhydroxyverbindung ist, welche bei Oxidation ein chinoides System oder ein Naphthalin-System bildet. Beispielsweise kann der organische Mediator ausgewählt sein aus der Gruppe enthaltend ortho-Dihydroxybenzol, para-Dihydroxybenzol, substituierte ortho-Dihydroxybenzole und substituierte para-Dihydroxybenzole, Dihydroxynaphtalin, substituiertes Dihydroxynaphtalin, Anthrahydrochinon, substituiertes Anthrahydrochinon, bevorzugt 1,2-Dihydroxybenzol, 1,4 Dihydroxybenzol, Naphtohydrochinon, substituiertes 1,2- oder 1,4.Dihydroxybenzol, substituiertes Hydrochinon, substituiertes Naphtohydrochinon, substituiertes Anthrahydrochinon, besonders bevorzugt substituiertes Hydrochinon, substituiertes 1,2-Dihydroxybenzol. Dabei ist es besonders günstig, wenn die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt und/oder tert-Butyl.

In einer weiteren Ausführungsform ist vorstellbar, dass die Gegenelektrode stabförmig ist. Die Gegenelektrode kann dabei zumindest teilweise aus der Absorptionsmittelzusammensetzung bestehen. Denkbar ist dabei weiterhin, dass die Arbeitselektrode die stabförmige Gegenelektrode tubusförmig umgibt. Beispielsweise kann die Arbeitselektrode einfach um die Gegenelektrode herum gewickelt sein. Denkbar ist auch, dass die Arbeitselektrode als Tubus ausgebildet und auf die stabförmige Gegenelektrode aufgeschoben ist. Günstig ist es dabei, wenn zwischen der Gegenelektrode und der Arbeitselektrode wenigstens eine Trennschicht, bevorzugt eine hydrophile und/oder elektrolytgetränkte Trennschicht, angeordnet ist.

Auch bei allen anderen denkbaren Ausführungsformen ist es vorstellbar, dass zwischen der Gegenelektrode und der Arbeitselektrode eine oder mehrere Trennschichten angeordnet sind. Die Trennschicht ist dabei bevorzugt ebenfalls hydrophil und/oder elektrolytgetränkt.

In jedem Fall kann die Trennschicht dabei in einer Fluidverbindung mit einem Elektrolytreservoir stehen. Beispielsweise kann der Gassensor derart ausgebildet sein, dass er einen Reaktionsraum, in welchem die Elektroden angeordnet sind, und ein Elektrolytreservoir, in welchem sich der flüssige Elektrolyt befindet, aufweist. Der Elektrolyt kann auf diese Weise mit Hilfe der Trennschicht vom Elektrolytreservoir zu den Elektroden geleitet werden. Die Trennschicht erstreckt sich dabei bevorzugt über den Reaktionsraum hinaus bis zum Elektrolytreservoir. Sie kann auf diese Weise eine Dochtwirkung aufweisen, durch welche der Elektrolyt zu den Elektroden transportiert wird. Die Trennschicht kann mithin eine Dochtwirkung haben. Mit anderen Worten, man erkennt dass die Trennschicht eine Elektrolytleitung zwischen dem Elektrolytreservoir und den Elektroden bilden kann. Um die Entfernung möglichst gering zu halten, über welche der Elektrolyt mit Hilfe der Dochtwirkung der Trennschicht transportiert wird, ist es denkbar, dass in der Trennschicht Elektrolytkanäle ausgebildet sind. Beispielsweise ist es vorstellbar, dass die Elektrolytkanäle seitlich parallel zu den von der Trennschicht bedeckten Elektroden ausgebildet sind.

Bei allen diesen Ausführungsbeispielen ist es auch vorstellbar, dass der Gassensor weiterhin eine Schutzelektrode aufweist. Dabei ist es besonders günstig, wenn die Schutzelektrode zwischen der Arbeitselektrode und der Gegenelektrode angeordnet ist. Die Schutzelektrode kann beispielsweise verhindern, dass an der Gegenelektrode entwickeltes Gas zurück zur Arbeitselektrode gelangen kann und dort zu Fehlsignalen führt. Zweckmäßig ist es dabei, wenn zwischen der Gegenelektrode und der Schutzelektrode eine diffusionsbegrenzende Trennschicht z.B in Form eines Glasfaservlieses angeordnet ist.

Weiterhin kann der Gassensor eine Referenzelektrode aufweisen. Beispielsweise kann eine flächig ausgebildete Referenzelektrode seitlich der Arbeitselektrode und/oder seitlich der Gegenelektrode angeordnet sein.

Alle denkbaren Ausführungsformen des erfindungsgemäßen Gassensors sind insbesondere geeignet zur Verwendung zum Nachweis von sauren Gasen und/oder Gasgemischen, welche saure Gase enthalten, bevorzugt zum Nachweis von HF, HCl und/oder Essigsäure. Dabei kann mit Hilfe der Absorptionsmittelzusammensetzung effektiv verhindert werden, dass das an der Gegenelektrode gebildete HF und/oder HCl freigesetzt werden kann. Die Verwendung eines entsprechenden Gassensors hat daher insbesondere den Vorteil, dass der Sensor dauerbegasungsfest ist. Ein solcher Sensor zeigt zurm ein gutes Abklingverhalten des Messsignals d.h. am Ende der Begasung zeigt der Sensor schnell wieder den Nullwert an. Das Auftreten von Drift des Messsignals bei Dauerbegasung ist weitestgehend verhinderbar. Weiterhin hat die Verwendung eines solchen erfindungsgemäßen Gassensors den Vorteil, dass aus dem Sensor kein giftiges und/oder ätzendes Gas freigesetzt wird.

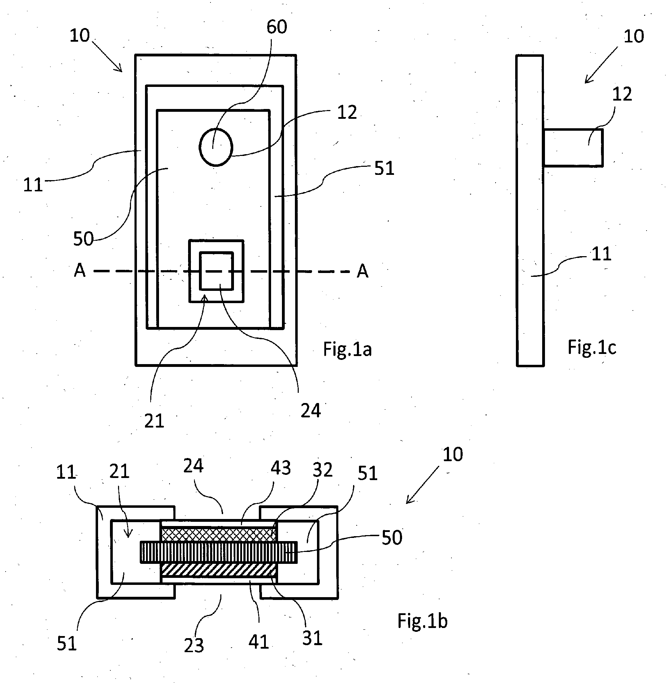

Weitere Merkmale, Details und Einzelheiten ergeben sich aus den nachfolgend beschriebenen Figuren und Ausführungsbeispielen. Es versteht sich von selbst, dass diese Ausführungsbeispiele nur exemplarisch sind und dass sich für den Fachmann anhand der vorliegenden Beschreibung ohne Probleme weitere Varianten und Ausführungsbeispiele ergeben. Dabei zeigen
- Fig. 1a bis 1c: einen schematischen Aufbau eines erfindungsgemäßen Gassensors, bei welchem die Gegenelektrode aus der Absorptionsmittelzusammensetzung besteht. Dabei zeigt Fig. 1a eine schematische Aufsicht auf den Gassensor, Fig. 1b einen Querschnitt durch den Reaktionsraum des Gassensors entlang der Linie A-A von Fig. 1a und Fig. 1c eine schematische Seitenansicht des Gassensors.

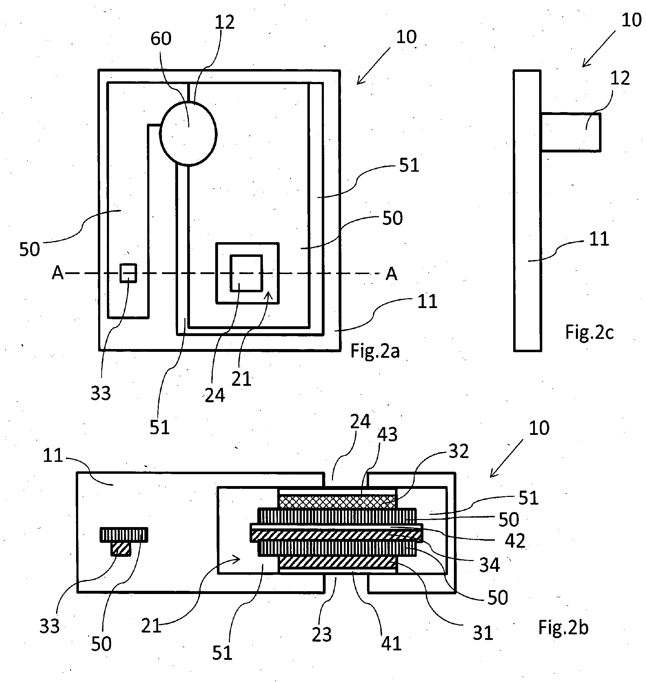

- Fig. 2a bis 2c: zeigen einen schematischen Aufbau eines erfindungsgemäßen Gassensors mit einer Hilfselektrode, die aus der Absorptionsmittelzusammensetzung besteht. Dabei zeigt Fig. 2a eine schematische Aufsicht auf den Gassensor, Fig. 2b einen Querschnitt durch den Reaktionsraum des Gassensors entlang der Linie A-A von Fig. 2a und Fig. 2c eine schematische Seitenansicht des Gassensors.

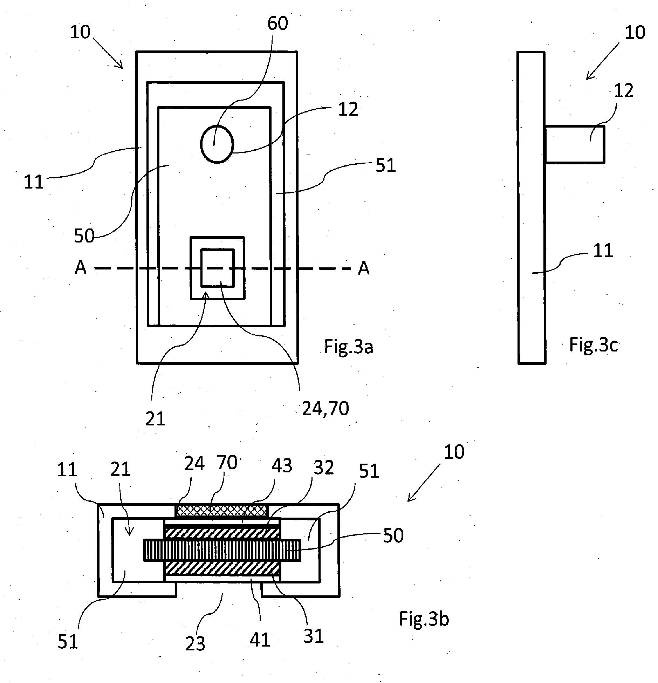

- Fig. 3a bis 3c: zeigen einen schematischen Aufbau eines erfindungsgemäßen Gassensors, bei welchem die Absorptionsmittelzusammensetzung einen Stopfen im Gasauslass des Gassensors bildet. Dabei zeigt Fig. 3a eine schematische Aufsicht auf den Gassensor, Fig. 3b einen Querschnitt durch den Reaktionsraum des Gassensors entlang der Linie A-A von Fig. 3a und Fig. 3c eine schematische Seitenansicht des Gassensors.

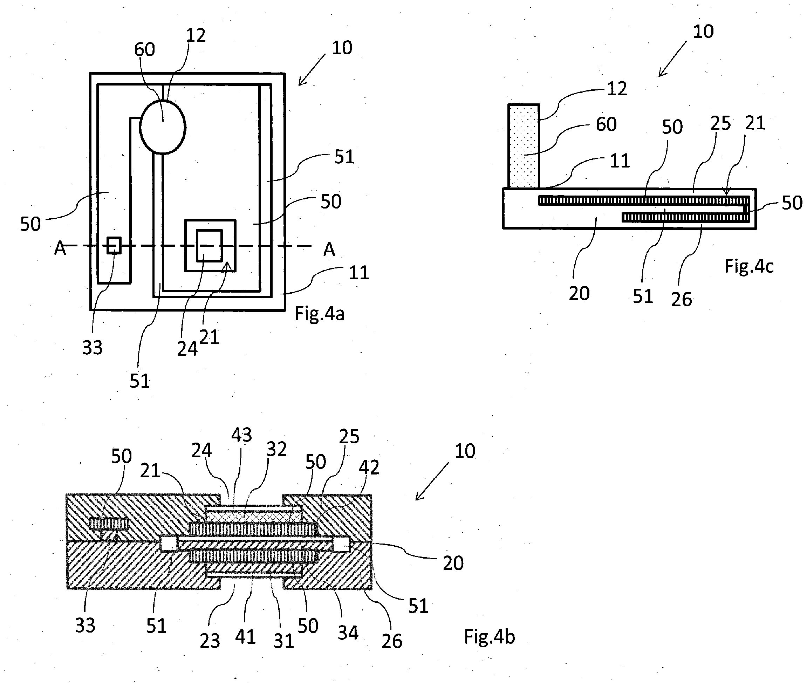

- Fig. 4a bis 4c: zeigen einen weiteren schematischen Aufbau eines erfindungsgemäßen Gassensor. Dabei zeigt Fig. 4a eine schematische Aufsicht auf den Gassensor, Fig. 4b einen Querschnitt durch den Reaktionsraum des Gassensors entlang der Linie A-A von Fig. 4a und Fig. 4c eine schematische Seitenansicht des Gassensors.

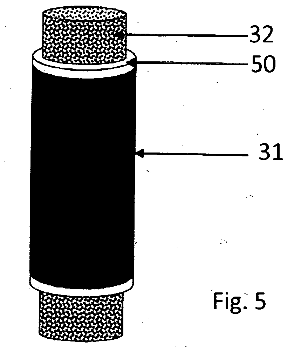

- Fig. 5: zeigt einen schematischen Aufbau einer erfindungsgemäßen Elektrodenanordnung, bei welcher eine aus der Absorptionsmittelzusammensetzung bestehende stabförmige Gegenelektrode mit einer Trennschicht und einer Arbeitselektrode umwickelt ist.

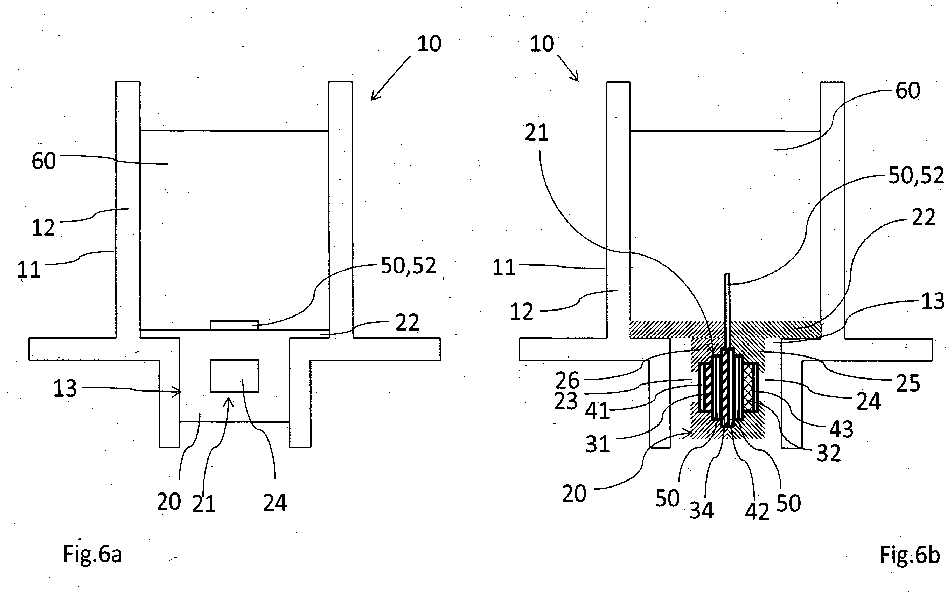

- Fig. 6a und 6b: zeigen einen alternativen schematischen Aufbau eines erfindungsgemäßen Gassensors. Dabei zeigt Fig. 6b eine um 90° gedrehte Ansicht des in Fig. 6a dargestellten Sensors.

Der in den Fig. 1a bis 1c, 2a bis 2c, 3a bis 3c, 4a bis 4c, 6a und 6b dargestellte Gassensor 10 hat ein Gehäuse 11 welches einen Reaktionsraum 21 umschließt. In dem Gehäuse 11 sind eine erste Ausnehmung 23 und eine zweite Ausnehmung 24 ausgebildet. Die erste Ausnehmung 23 ist ein Gaseinlass durch welchen zu analysierendes Gas in den Reaktionsraum 21 einströmen kann. Die zweite Ausnehmung 24 ist ein Gasauslass, durch welchen an der Gegenelektrode 32 entstehendes Gas aus dem Reaktionsraum 21 ausströmen kann. In dem Reaktionsraum 21 sind die Elektroden 31, 32 des Gassensors 10 angeordnet, nämlich die Arbeitselektrode 31 und die Gegenelektrode 32. Zwischen der Arbeitselektrode 31 und der Gegenelektrode 32 befindet sich eine Trennschicht 50. Die Trennschicht 50 ist hydrophil.

Weiterhin befindet sich zwischen der Arbeitselektrode 31 und der ersten Ausnehmung 23 eine hydrophobe Membran 41. Eine weitere hydrophobe Membran 43 befindet sich zwischen der Gegenelektrode 32 und der zweiten Ausnehmung 24. Die beiden hydrophoben Membranen 41, 43 verhindern, dass Elektrolyt 60 aus dem Reaktionsraum 21 austritt, und schützen gleichzeitig die den Ausnehmungen 23, 24 gegenüberliegenden Elektroden 31, 32 vor Staub und Schmutz, die andernfalls möglicherweise durch die Ausnehmungen 23, 24 in den Reaktionsraum 21 eingetragen werden könnten.

Der Gassensor 10 hat weiterhin ein Elektrolytreservoir 12, in welchem sich der flüssige Elektrolyt 60 befindet. Man erkennt, dass die Trennschicht 50 das Elektrolytreservoir 12 mit demReaktionsraum 21 verbindet. Die Trennschicht 50 ist dabei mit dem flüssigen Elektrolyten 60 getränkt. Auf diese Weise steht der Reaktionsraum 21 in fluider Verbindung mit dem Elektrolytreservoir 12. Seitlich der Trennschicht 50 sind Elektrolytkanäle 51 ausgebildet. Diese dienen der Unterstützung der Fluidverbindung zwischen dem Elektrolytreservoir 12 und dem Reaktionsraum 21.

Der in den Figuren 2a bis 2c und 4a bis 4c dargestellte Gassensor 10 weist weiterhin eine Referenzelektrode 33 auf. Man erkennt, dass auch die Referenzelektrode 33 über die Trennschicht 50 mit dem Elektrolytreservoir 12 verbunden ist. Wie man darüber hinaus sehen kann, ist die Referenzelektrode 33 in den gezeigten Beispielen von der Trennschicht 50 bedeckt. In einer nicht dargestellten Variante ist jedoch auch vorstellbar, dass die Referenzelektrode 33 in die Trennschicht 50 eingebettet ist. In jedem Fall steht auch die Referenzelektrode 33 in Fluidverbindung mit dem Elektrolytreservoir 12, so dass der Elektrolyt 60 mit Hilfe der Trennschicht 50 von dem Elektrolytreservoir 12 zu der Referenzelektrode 33 geleitet wird. In der Aufsicht auf der Figur 2a wird deutlich, dass die Referenzelektrode 33 in seitlichem Abstand zu den im Reaktionsraum 21 angeordneten Elektroden 31, 32, 34 angeordnet ist. Gleichzeitig stehen jedoch alle Elektroden des Gassensors 10 durch den Elektrolyten 60 in einem leitenden Kontakt miteinander.

Der beispielhaft in den Figuren 2a bis 2c und 4a bis 4c dargestellte Gassensor 10 hat zusätzlich zu den bereits beschriebenen Elektroden 31, 32, 33 eine zwischen der Arbeitselektrode 31 und der Gegenelektrode 32 angeordnete Schutzelektrode 34. Man erkennt, dass die Elektroden 31, 32, 34 dabei sandwichartig angeordnet sind. Über der Arbeitselektrode 31 ist die Trennschicht 50 angeordnet. Man erkennt, dass die Trennschicht 50 die Arbeitselektrode 31 vollständig bedeckt. Über der Trennschicht 50 ist die Schutzelektrode 34 angeordnet. Die Schutzelektrode 34 ist von einer Membran 42 bedeckt. Die Membran 42 ist in einer speziellen Variante eine diffusionsbegrenzende Membran. Oberhalb der Membran 42 ist eine weitere Schicht der Trennschicht 50 ausgebildet. Auf dieser Trennschicht 50 ist die Gegenelektrode 32 angeordnet. Bei dieser Anordnung der Elektroden 31, 32, 34 ist die Schutzelektrode 34 derart ausgebildet, dass die Fläche der Schutzelektrode 34 größer als die Fläche der Arbeitselektrode 31 und größer als die Fläche der Gegenelektrode 32 ist.

In den in den Fig. 4a bis 4c, sowie 6a und 6b gezeigten Ausführungsform wird das Gehäuse 11 von einem Elektrolytreservoir 12 und einem Elektrodenträger 20 gebildet. Der dem Betrachter zugewandte Teil des Gehäuses 11 ist in der in Fig. 4a dargestellten Ansicht insbesondere im Bereich des Elektrodenträgers 20 transparent dargestellt, um zu verdeutlichen, wie die Anordnung der nachfolgend beschriebenen Bauteile im Inneren des Elektrodenträgers 20 gestaltet sein kann.

Auch hier ist in dem Elektrolytreservoir 12 ein flüssiger Elektrolyt 60 vorhanden. Der Elektrodenträger 20 weist einen Reaktionsraum 21 auf. In den Reaktionsraum 21 kann durch eine (in Fig. 4b und 6b erkennbare) erste Ausnehmung 23 Gas eintreten und durch eine zweite Ausnehmung 24 Gas austreten. Die erste Ausnehmung 23 ist dabei in dem gezeigten Beispiel an der Unterseite des Elektrodenträgers 20 ausgebildet, während die zweite Ausnehmung 24 in der gegenüberliegenden Oberseite des Elektrodenträgers 20 ausgebildet ist. Es versteht sich von selbst, dass es alternativ auch denkbar ist, dass die erste Ausnehmung 23 in der Oberseite und die zweite Ausnehmung 24 in der Unterseite des Elektrodenträgers 20 ausgebildet ist. Man erkennt mithin, dass der Elektrodenträger 20 wenigstens eine erste Ausnehmung 23 aufweist.

Der Reaktionsraum 21 ist auch hier über eine Trennschicht 50 mit dem Elektrolytreservoir 12 verbunden, so dass der Reaktionsraum 21 über die Trennschicht 50 mit dem Elektrolytreservoir 12 in einer Fluidverbindung steht. Um die Zuleitung des Elektrolyten 60 noch zu verbessern, sind seitlich der Trennschicht 50 Elektrolytkanäle 51 ausgebildet. Es ist selbstverständlich auch vorstellbar, dass nur ein Elektrolytkanal 51 ausgebildet ist oder dass mehr als zwei Elektrolytkanäle 51 vorhanden sind.

Man erkennt sowohl in Fig. 4b als auch in Fig. 4c, sowie in Fig. 6b, dass der Reaktionsraum 21 von einem ersten Wandabschnitt 25 und einem zweiten Wandabschnitt 26 des Elektrodenträgers 20 begrenzt wird. Mithin formt der Elektrodenträger 20 den Reaktionsraum 21. Dabei sind die Elektroden 31, 32, 34 zwischen dem ersten Wandabschnitt 25 und dem zweiten Wandabschnitt 26 angeordnet. Weiterhin erkennt man, dass der Reaktionsraum 21 über die erste und die zweite Ausnehmung 23, 24 mit der Umgebungsluft verbunden ist. In Fig. 4c erkennt man weiterhin, dass der Elektrodenträger 20 einen Abschnitt des Gehäuses 11 des Gassensors 10 bildet. Das Gehäuse 11 besteht dabei aus der Wand des Elektrolytreservoirs 12 und aus der Wand des Elektrodenträgers 20. Die Trennschicht 50 ist derart in dem Elektrodenträger 20 angeordnet, dass sie in direktem Kontakt mit dem in dem Elektrolytreservoir 12 vorhandenen Elektrolyten 60 steht.

Auch bei der in den Fig. 6a und 6b dargestellten alternativen Ausführungsform des Gassensors 10 ist die Trennschicht 50, insbesondere ein aus dem Elektrodenträger 20 herausragender Abschnitt 52 der Trennschicht 50, in direktem Kontakt mit dem in dem Elektrolytreservoir 12 vorhandenen Elektrolyten 60. Man erkennt auch hier, dass der Elektrodenträger 20 einen Abschnitt des Gehäuses 11 bildet. Dabei weist der Elektrodenträger 20 einen Befestigungsabschnitt 22 auf. Mit diesem Befestigungsabschnitt 22 ist der Elektrodenträger 20 in einer Aufnahme 13 des Elektrolytreservoirs 12 festgelegt. Dabei ist der Elektrodenträger 20 derart angeordnet, dass derjenige Teil des Elektrodenträgers 20, in welchem der Reaktionsraum 21 ausgebildet ist, einen Abschnitt des Gehäuses 11 bildet. Der Elektrodenträger 20 hat in diesem Bereich eine innere Oberfläche und eine äußere Oberfläche 28. Die innere Oberfläche begrenzt den Reaktionsraum 21. Die äußere Oberfläche 28 bildet einen Abschnitt der äußeren Gehäusewand des Gassensors 10.

Bei den in den Figuren 1a bis 1c und 2a bis 2c dargestellten Ausführungsbeispielen besteht jeweils die Gegenelektrode 32 aus der Absorptionsmittelzusammensetzung70. In einer ersten Variante besteht die Absorptionsmittelzusammensetzung70 dabei aus einer Mischung aus BaCO₃ als Absorptionsmittel, zerkleinerten Glasfasern als Trägermaterial und Kohlenstoffnanoröhren als Zusatzstoff.

Bei dem in den Figuren 3a bis 3b dargestellten Gassensor 10 ist in der Ausnehmung 24, die den Gasauslass bildet, die Absorptionsmittelzusammensetzung 70 in Form eines Stopfens angeordnet. Aus dem Gassensor 10 ausströmendes Gas muss dabei durch die Ausnehmung 24 hindurch strömen, also durch den Gasauslass, und mithin durch die Absorptionsmittelzusammensetzung 70 hindurch. Die Absorptionsmittelzusammensetzung 70 besteht dabei in einer ersten Variante aus einer Zusammensetzung, die BaCO₃ als Absorptionsmittel, Glasfasern als Trägermaterial und Teflonfasern als Zusastzstoff enthält. In einer zweiten Variante besteht die Absorptionsmittelzusammensetzung 70 aus CaCO₃ als Absorptionsmittel, Glasfasern als Trägermaterial und Teflonfasern als Zusatzstoff. Alternativ ist auch denkbar, dass ein anderes Alkali- oder Erdalkalicarbonat als Absorptionsmittel in der Absorptionsmittelzusammensetzung 70 enthalten ist.

In einer nicht dargestellten Ausführungsform ist der Gassensor 10 wie für die Figuren 1a bis 1c beschrieben aufgebaut. Bei dieser Ausführungsform kann zusätzlich zu der aus einer ersten Absorptionsmittelzusammensetzung bestehenden Gegenelektrode 32 auch noch in der Ausnehmung 24, die den Gasauslass bildet, Absorptionsmittelzusammensetzung 70 in Form eines Stopfens angeordnet sein. Die Absorptionsmittelzusammensetzung 70 weist in einer ersten Variante die gleiche Zusammensetzung auf, wie die erste Absorptionsmittelzusammensetzung, aus welcher die Gegenelektrode 32 besteht. Dabei enthält die Absorptionsmittelzusammensetzung BaCO₃ als Absorptionsmittel, Glasfasern als Trägermittel und Kohlenstoffnanoröhren als Zusatzstoff. In einer zweiten Variante hat die Absorptionsmittelzusammensetzung 70 eine andere Zusammensetzung als die Absorptionsmittelzusammensetzung, aus welcher die Gegenelektrode 32 besteht. Bei dieser Variante enthält die Absorptionsmittelzusammensetzung, aus welcher die Gegenelektrode 32 besteht, BaCO₃ als Absorptionsmittel, Glasfasern als Trägermittel und Kohlenstoffnanoröhren als Zusatzstoff. Die Absorptionsmittelzusammensetzung 70, die in der Ausnehmung 24, also im Gasauslass, angeordnet ist enthält BaCO₃ oder in weiteren Varianten ein anderes Alkali- oder Erdalkalicarbonat als Absorptionsmittel, Glasfasern als Trägermaterial und Teflonfasern als Zusastzstoff.

In einer weiteren nicht dargestellten Ausführungsform ist der Gassensor 10 entsprechend den Figuren 2a bis 2c aufgebaut wobei zusätzlich in der Ausnehmung 24, die den Gasauslass bildet, Absorptionsmittelzusammensetzung 70 angeordnet ist. Dabei weist die Absorptionsmittelzusammensetzung 70 in einer ersten Variante die gleiche Zusammensetzung auf, wie die Absorptionsmittelzusammensetzung, aus welcher die Gegenelektrode 32 besteht. Dabei enthält die Absorptionsmittelzusammensetzung - wie bereits für die Varianten der in den Fig. 1a bis 1c dargestellten Ausführungsform - BaCO₃ als Absorptionsmittel, Glasfasern als Trägermittel und Kohlenstoffnanoröhren als Zusatzstoff. In einer zweiten Variante hat die Absorptionsmittelzusammensetzung 70 eine andere Zusammensetzung als die Absorptionsmittelzusammensetzung, aus welcher die Gegenelektrode 32 besteht. Auch bei dieser Variante enthält die Absorptionsmittelzusammensetzung, aus welcher die Gegenelektrode 32 besteht, BaCO₃ als Absorptionsmittel, Glasfasern als Trägermittel und Kohlenstoffnanoröhren als Zusatzstoff. Die Absorptionsmittelzusammensetzung 70, die in der Ausnehmung 24, also im Gasauslass, angeordnet ist enthält BaCO₃ oder in weiteren Varianten ein anderes Alkali- oder Erdalkalicarbonat als Absorptionsmittel, Glasfasern als Trägermaterial und Teflonfasern als Zusatzstoff.

In noch einer weiteren nicht dargestellten Ausführungsform ist der Gassensor 10 entsprechend den Figuren 3a bis 3c aufgebaut wobei nicht nur in der Ausnehmung 24, die den Gasauslass bildet, Absorptionsmittelzusammensetzung 70 angeordnet ist, sondern auch die Gegenelektrode 32 aus Absorptionsmittelzusammensetzung besteht. Dabei weist die

Absorptionsmittelzusammensetzung 70 in einer ersten Variante die gleiche Zusammensetzung auf, wie die Absorptionsmittelzusammensetzung, aus welcher die Gegenelektrode 32 besteht. In einer zweiten Variante hat die Absorptionsmittelzusammensetzung 70 eine andere Zusammensetzung als die Absorptionsmittelzusammensetzung, aus welcher die Gegenelektrode 32 besteht. Die jeweiligen Absorptionsmittelzusammensetzungen können in beiden Varianten wie bereits oben beschrieben zusammengesetzt sein.

Auch bei den in den Figuren 4a bis 4c und 6a sowie 6b dargestellten Ausführungsformen des Gassensors 10 besteht die Gegenelektrode 32 aus der Absorptionsmittelzusammensetzung. In einer ersten nicht dargestellten Variante ist auch hier zusätzlich in der Ausnehmung 24, die den Gasauslass bildet, Absorptionsmittelzusammensetzung 70 in Form eines Stopfens angeordnet. Dabei ist die Absorptionsmittelzusammensetzung 70, die im Gasauslass, d.h. in der Ausnehmung 24, angeordnet ist, die gleiche wie die Absorptionsmittelzusammensetzung aus der die Gegenelektrode 32 besteht. Auch in einer zweiten nicht dargestellten Variante ist zusätzlich in der Ausnehmung 24, die den Gasauslass bildet, Absorptionsmittelzusammensetzung 70 in Form eines Stopfens angeordnet. Dabei unterscheidet sich jedoch die Zusammensetzung der in dem Gasauslass, d.h. in der Ausnehmung 24, angeordneten Absorptionsmittelzusammensetzung 70 jedoch von der Zusammensetzung der Absorptionsmittelzusammensetzung aus welcher die Gegenelektrode 32 besteht.

Eine besondere Ausführungsvariante für die Anordnung der Arbeits- und der Gegenelektrode 31, 32 erkennt man in Fig. 5. Hier ist die Gegenelektrode 32 aus der Absorptionsmittelzusammensetzung geformt und hat stabförmige Gestalt. Die Arbeitselektrode 31 ist in Form eines Tubus (tubusförmig) ausgebildet und auf die Gegenelektrode 32 aufgesteckt. Zwischen der Arbeitselektrode 31 und der Gegenelektrode 32 ist die Trennschicht 50 ausgebildet. In einer alternativen Ausführungsform, kann die Gegenelektrode 32 auch die Form eines Tubus haben (tubusförmig).

Ein Beispiel für eine Zusammensetzung eines Elektrolyten, wie er zusammen mit den oben beschriebenen Ausführungsbeispielen verwendbar ist, ist eine Mischung aus etwa 60 Gewichtsprozent Propylencarbonat, etwa 40 Gewichtsprozent Ethylencarbonat, etwa 0,1 mol HMIM FAP (1-Hexyl-3-methyl-imidazolium-tris(pentafluoroethyl)-trifluorophosphat) und etwa 0,5 mol tert-Butylhydrochinon. Diese Zusammensetzung ist selbstverständlich variierbar, so ist das Verhältnis von Proplyencarbonat zu Ethylencarbonat keineswegs auf ein Verhältnis von 60:40 Gewichtsprozent beschränkt. Auch die molare Menge von enthaltenem HMIM FAP und tert-Butylhydrochinon ist variierbar.

**Bezugszeichenliste**

| | | | |
|---|---|---|---|
| A | Linie | 31 | Arbeitselektrode |
| | | 32 | Gegenelektrode |
| 10 | Gassensor | 33 | Referenzelektrode |
| 11 | Gehäuse | 34 | Schutzelektrode |
| 12 | Elektrolytreservoir | | |
| 13 | Aufnahme | 41 | Membran |
| | | 42 | Membran |
| 20 | Elektrodenträger | 43 | Membran |
| 21 | Reaktionsraum | | |
| 22 | Befestigungsabschnitt | 50 | Trennschicht |
| 23 | Ausnehmung | 51 | Elektrolytkanal |
| 24 | Ausnehmung | 52 | Abschnitt |
| 25 | Wandabschnitt | | |
| 26 | Wandabschnitt | 60 | Elektrolyt |
| 27 | Oberfläche | | |
| 28 | Oberfläche | 70 | Absorptionsmittelzusammensetzung |

## Patentansprüche

1. Elektrochemischer Gassensor (10) mit einem Gehäuse (11), mit mehreren darin angeordneten Elektroden (31, 32), nämlich wenigstens einer Arbeitselektrode (31) und einer Gegenelektrode (32), sowie mit einem flüssigen Elektrolyten (60), wobei wenigstens die Gegenelektrode (32) und/oder das Gehäuse (11) zumindest teilweise aus einer Absorptionsmittelzusammensetzung (70) besteht, wobei die Absorptionsmittelzusammensetzung (70) BaCO₃ als Absorptionsmittel enthält, wobei das Gehäuse (11) eine Ausnehmung aufweist, die einen Gaseinlass (23) bildet, und eine Ausnehmung (24) aufweist, die einen Gasauslass bildet, wobei die Absorptionsmittelzusammensetzung (70) ganz oder teilweise in der Ausnehmung (24) angeordnet ist und wobei die Absorptionsmittelzusammensetzung (70), die in der Ausnehmung (24) angeordnet ist die Form eines Stopfens aufweist.

2. Gassensor nach Anspruch 1, **dadurch gekennzeichnet, dass** die Absorptionsmittelzusammensetzung wenigstens ein Absorptionsmittel, ein Trägermaterial und einen Zusatzstoff aufweist.

3. Gassensor nach einem der Ansprüche 1 oder 2, **dadurch gekennzeichnet, dass** die Absorptionsmittelzusammensetzung Kohlenstoffnanoröhren als Zusatzstoff aufweist.

4. Gassensor nach einem der Ansprüche 1 bis 3, **dadurch gekennzeichnet, dass** die Absorptionsmittelzusammensetzung ein Absorptionsmittel enthält, das schwer löslich oder unlöslich in dem Elektrolyt ist.

5. Gassensor nach einem der Ansprüche 1 bis 4, **dadurch gekennzeichnet, dass** die Absorptionsmittelzusammensetzung ein faserförmiges Material, bevorzugt ein mikrofaserförmiges Material, besonders bevorzugt Glasfasern, Mikro- und/oder Nanofasern, bevorzugt Polymer-Mikro- und/oder Nanofasern, als Trägermaterial enthält.

6. Gassensor nach einem der Ansprüche 1 bis 5, **dadurch gekennzeichnet, dass** die Absorptionsmittelzusammensetzung ein Teflonmaterial als Zusatzstoff aufweist.

7. Gassensor nach einem der Ansprüche 1 bis 6, **dadurch gekennzeichnet, dass** der Elektrolyt eine Zusammensetzung ist, die ein organisches Lösungsmittel und ein Leitsalz enthält, bevorzugt eine Zusammensetzung, die ein organisches Lösungsmittel, welches ein chinoides System enthält, und ein Leitsalz, welches ein organisches Kation aufweist, enthält.

8. Gassensor nach einem der Ansprüche 1 bis 7, **dadurch gekennzeichnet, dass** die Gegenelektrode (32) stabförmig ist.

9. Gassensor nach Anspruch 8, **dadurch gekennzeichnet, dass** die Arbeitselektrode (31) die stabförmige Gegenelektrode (32) tubusförmig umgibt.

10. Gassensor nach einem der Ansprüche 1 bis 9, **dadurch gekennzeichnet, dass** zwischen der Gegenelektrode (32) und der Arbeitselektrode (31) wenigstens eine Trennschicht (50), bevorzugt eine hydrophile und/oder elektrolytgetränkte Trennschicht (50), angeordnet ist.

11. Gassensor nach einem der Ansprüche 1 bis 10, **dadurch gekennzeichnet, dass** der Gassensor weiterhin eine Schutzelektrode (34) und/oder eine Referenzelektrode (33) aufweist.

12. Verwendung eines elektrochemischen Gassensors (10) entsprechend einem der Ansprüche 1 bis 11 zum Nachweis von sauren Gasen und/oder Gasgemischen, welche saure Gase enthalten, bevorzugt zum Nachweis von HF, HCl und/oder Essigsäure

## Claims

1. Electrochemical gas sensor (10) having a housing (11), having a plurality of electrodes (31, 32) arranged therein, namely at least a working electrode (31) and a counter electrode (32), and having a liquid electrolyte (60), wherein at least the counter electrode (32) and/or the housing (11) are composed at least partially of an absorbent composition (70), wherein the absorbent composition (70) contains BaCO₃ as absorbent, wherein the housing (11) has an opening that forms a gas inlet (23), and an opening (24) that forms a gas outlet, wherein the absorbent composition (70) is arranged wholly or partially in the opening (24) and wherein the absorbent composition (70) that is arranged in the opening (24) has the form of a stopper.

2. Gas sensor according to Claim 1, **characterized in that** the absorbent composition includes at least an absorbent, a carrier material and an additive.

3. Gas sensor according to either of Claims 1 and 2, **characterized in that** the absorbent composition includes carbon nanotubes as additive.

4. Gas sensor according to any of Claims 1 to 3, **characterized in that** the absorbent composition contains an absorbent which is sparingly soluble or insoluble in the electrolyte.

5. Gas sensor according to any of Claims 1 to 4, **characterized in that** the absorbent composition contains a fibrous material, preferably a microfibrous material, particularly preferably glass fibres, micro- and/or nanofibres, preferably polymer micro- and/or nanofibres, as carrier material.

6. Gas sensor according to any of Claims 1 to 5, **characterized in that** the absorbent composition includes a Teflon material as additive.

7. Gas sensor according to any of Claims 1 to 6, **characterized in that** the electrolyte is a composition which contains an organic solvent and a conductive salt, preferably a composition which contains an organic solvent containing a quinoid system and a conductive salt comprising an organic cation.

8. Gas sensor according to any of Claims 1 to 7, **characterized in that** the counter electrode (32) is rod-shaped.

9. Gas sensor according to Claim 8, **characterized in that** the working electrode (31) surrounds the rod-shaped counter electrode (32) in the form of a tube.

10. Gas sensor according to any of Claims 1 to 9, **characterized in that**, between the counter electrode (32) and the working electrode (31) there is arranged at least one separating layer (50), preferably a hydrophilic and/or electrolyte-impregnated separating layer (50).

11. Gas sensor according to any of Claims 1 to 10, **characterized in that** the gas sensor further includes a protective electrode (34) and/or a reference electrode (33) .

12. Use of an electrochemical gas sensor (10) in accordance with any of Claims 1 to 11 for detection of acidic gases and/or gas mixtures containing acidic gases, preferably for detection of HF, HCl and/ or acetic acid.

## Revendications

1. Capteur de gaz électrochimique (10) comprenant un boîtier (11), une pluralité d'électrodes (31, 32) disposées dans ledit boîtier, à savoir au moins une électrode de travail (31) et une contre-électrode (32), et un électrolyte liquide (60), au moins la contre-électrode (32) et/ou le boîtier (11) étant au moins partiellement en une composition d'agent d'absorption (70), la composition d'agent d'absorption (70) contenant du BaCO₃ comme agent d'absorption, le boîtier (11) comportant un évidement, qui comporte une entrée de gaz (23), et un évidement (24) qui forme une sortie de gaz, la composition d'agent d'absorption (70) étant disposée en totalité ou en partie dans l'évidement (24) et la composition d'agent d'absorption (70), qui est disposée dans l'évidement (24), ayant la forme d'un bouchon.

2. Capteur de gaz selon la revendication 1, **caractérisé en ce que** la composition d'agent d'absorption comporte au moins un agent d'absorption, un matériau porteur et un additif.

3. Capteur de gaz selon l'une des revendications 1 ou 2, **caractérisé en ce que** la composition d'agent d'absorption comporte des nanotubes de carbone comme additif.

4. Capteur de gaz selon l'une des revendications 1 à 3, **caractérisé en ce que** la composition d'agent d'absorption contient un agent d'absorption peu soluble ou insoluble dans l'électrolyte.

5. Capteur de gaz selon l'une des revendications 1 à 4, **caractérisé en ce que** la composition d'agent d'absorption contient un matériau fibreux, de préférence un matériau micro-fibreux, de manière particulièrement préférée des fibres de verre, des microfibres et/ou des nano-fibres, de préférence des microfibres et/ou des nano-fibres polymères, comme matériau porteur.

6. Capteur de gaz selon l'une des revendications 1 à 5, **caractérisé en ce que** la composition d'agent d'absorption comporte un matériau Téflon comme additif.

7. Capteur de gaz selon l'une des revendications 1 à 6, **caractérisé en ce que** l'électrolyte est une composition qui contient un solvant organique et un sel conducteur, de préférence une composition qui contient un solvant organique, contenant un système quinoïde, et un sel conducteur comportant un cation.

8. Capteur de gaz selon l'une des revendications 1 à 7, **caractérisé en ce que** la contre-électrode (32) est en forme de tige.

9. Capteur de gaz selon la revendication 8, **caractérisé en ce que** l'électrode de travail (31) entoure en forme de tube la contre-électrode (32) en forme de tige.

10. Capteur de gaz selon l'une des revendications 1 à 9, **caractérisé en ce qu'**au moins une couche séparatrice (50), de préférence une couche séparatrice (50) hydrophile et/ou imprégnée d'électrolyte, est disposée entre la contre-électrode (32) et l'électrode de travail (31) .

11. Capteur de gaz selon l'une des revendications 1 à 10, **caractérisé en ce que** le capteur de gaz comporte également une électrode de protection (34) et/ou une électrode de référence (33).

12. Utilisation d'un capteur de gaz électrochimique (10) selon l'une des revendications 1 à 11 pour la détection de gaz acides et/ou de mélanges gazeux contenant des gaz acides, de préférence pour la détection de HF, HCl et/ou d'acide acétique.
